# EUROPEAN PATENT APPLICATION

(11) **EP 2 363 116 A1**
(43) Date of publication of application: **07.09.2011**
(21) Application number: 11002224.1
(22) Date of filing: 25.06.2009
(51) Int. Cl.: A61K 9/20, A61K 9/70

(54) **Sustained-release formulations comprising lofexidine for oral delivery**

(30) Priority: 25.06.2008 US 75550
(62) Divisional of application: 09771009.9
(71) Applicant: US Worldmeds LLC, Louisville, KY 40207 (US)
(72) Inventor: Al-Ghananeem, Abeer M., Lexington, KY 40513 (US)
(74) Representative: Samson & Partner

(57) **Abstract**

This invention relates to a sustained release oral pharmaceutical formulations and delivery systems comprising lofexidine. The invention is also directed to methods of treatment comprising administering lofexidine in a sustained release manner. Such methods can involve administration of the lofexidine containing compositions described herein. Compositions of lofexidine formulated for sustained release delivery are provided. Also provided are methods for the treatment of opiate addicts, migraine, neuropathic pain, and other therapeutic indications related to lofexidine. The methods may provide treatment for a variety of conditions amenable to amelioration by lofexidine administration. The methods utilize lofexidine compositions formulated for sustained release oral delivery for administration of lofexidine in an amount effective for the treatment of the drug indications.

## Description

### CROSS-REFERENCE TO RELATED APPLICATIONS

This application claims priority to U.S. Provisional Patent Application Serial. No. 61/075,550, which was filed on June 25, 2008, the entirety of which is incorporated herein by reference for all purposes.

### FIELD OF THE INVENTION

The present invention overcomes the problems associated with existing immediate release drug delivery systems of lofexidine by delivering the therapeutic agent transdermally or through sustained release oral formulations. In one embodiment, the lofexidine is delivered via oral sustained release tablet formulations or an occlusive body *(i.e.,* a patch) to alleviate harmful side effects and avoid gastrointestinal (first-pass) metabolism of the drug by the patient.

The present invention relates to formulations for transdermal and oral delivery of lofexidine. The present invention also relates to compositions and methods for sustained release and combination sustained release/rapid release oral and transdermal formulations comprising lofexidine formulated in capsules, oral tablets, transdermal formulations, or a patch-needle hybrid (Microneedle) to deliver lofexidine by itself or in combination with other medications for many therapeutic uses, including but not restricted to: opiate detoxification, alcohol withdrawal syndrome, decrease stress-induced reinstatement of seeking addictive materials, pain management such as neuropathic pain and migraine, alleviate tobacco and alcohol withdrawal symptoms, treat intraocular pressure (IOP), anti-diarrheal agent, treat cardiovascular complications in patients with obstructive sleep apnea, and to prevent adverse effects of N-methyl-D-aspartate (NMDA) antagonists or schizophrenia-associated NMDA receptor hypofunction, and other conditions amenable to amelioration by lofexidine administration.

### BACKGROUND OF THE INVENTION

Lofexidine is an α2-adrenergic receptor agonist analogue of clonidine that acts centrally to suppress opiate withdrawal symptoms. The drug has been available for use as a non-opioid medication for opioid detoxification in the United Kingdom under the label BritLofex since 1992. Lofexidine was reported to be metabolized after oral delivery more extensively than the related anti-hypertensive agent, clonidine. The principal metabolite of lofexidine was reported to be 2,6-dichlorophenol, which was apparently excreted in urine as two *O*-glucuronic acid conjugates.

Effective drug therapies require control of blood serum levels of the drug. Sustained release pharmaceutical formulations provide a significant advantage over immediate release formulations to both clinicians and their patients. Sustained release dosage formulations are administered to patients in much fewer daily doses than their immediate release counterparts and generally achieve improved therapeutic effect and efficiency in the fewer daily doses.

Besides reducing the frequency of dosing and providing a more consistent therapeutic effect, sustained release dosage forms generally help reduce side effects caused by a drug. The reduction in side effects is primarily due to the consistent blood serum levels of the drug due to the slow, incremental release of sustained release dosage formulations. In contrast, immediate release formulations result in drug concentration highs and lows, or "peaks and troughs," relative to ideal concentration target levels.

In another aspect, the transdermal formulation is uniformly released in the therapy due to its stable permeation rate to the skin for the maintenance of the drug effective concentration in the blood. Should the patients feel uncomfortable; the therapeutic process can be discontinued immediately without the interference of the remaining drug in the body. Therefore, the convenience in usage increases the patient cooperation factor. Moreover, the likelihood of illness recurrence caused by lapses in taking medication, such as through forgetfulness, can be diminished

Currently, lofexidine is available as an immediate release tablets (0.2 mg) with a dosing regimen that requires multi-tablets to be given three to four times daily for few days. For patients who self-administer therapies, sustained release dosage forms generally result in greater compliance due to the lower frequency of dosing, lower quantity of dosage units to be consumed, and reduced undesired side-effects.

For the foregoing reasons, there is a need for drug formulations, such as sustained release drug formulations, such as sustained release oral and transdermal formulations, which are capable of stable therapeutic dosage profiles by providing an extended serum level concentration of lofexidine for an extended period in order to avoid possible "peak and trough" side effects.

The present invention now provides transdermal and sustained release oral delivery of lofexidine. In addition, the present invention provides for methods and compositions for oral sustained release delivery of lofexidine. Also, a method and mode of transdermally delivering lofexidine to treat various illnesses and/or symptoms are provided.

### BRIEF SUMMARY OF THE INVENTION

The present invention overcomes the problems and disadvantages associated with current immediate release dosage forms of lofexidine by delivering the therapeutic agent transdermally or through sustained release oral formulations. In one embodiment, the lofexidine is delivered via oral sustained release tablet formulations or an occlusive body (i.e., a patch) for transdermal delivery to alleviate harmful side effects and avoid gastrointestinal (first-pass) metabolism of the drug by the patient.

The invention also provides for materials and methods for sustained release oral and transdermal pharmaceutical formulations and delivery systems comprising lofexidine.

In one embodiment, the present invention to provide a process for producing such sustained release pharmaceutical oral and transdermal formulations. In one embodiment, the oral sustained release formulations comprising tablets or capsules. In another embodiment, the transdermal formulations comprising a skin patch or a patch-needle hybrid (Microneedle).

In another embodiment, the present invention to provide a combination sustained release/rapid release pharmaceutical oral and transdermal formulations.

In another embodiment, the invention provides methods of treatment comprising administering lofexidine in a sustained release manner. In one embodiment, the methods can involve administration of the lofexidine containing compositions described herein. In another embodiment, the methods may provide treatment for a variety of conditions amenable to amelioration by lofexidine administration.

In another embodiment, the present invention provides a pharmaceutical composition comprising lofexidine or a pharmaceutically acceptable salt thereof and a pharmaceutically acceptable carrier, wherein the lofexidine or pharmaceutically acceptable salt thereof is provided in a form suitable for oral or transdermal administration.

These and other embodiments of the invention are described herein below or are evident to persons of ordinary skill in the art based on the following disclosures.

The above summary of the present invention is not intended to describe each embodiment or every implementation of the present invention. Advantages and attainments, together with a more complete understanding of the invention, will become apparent and appreciated by referring to the following detailed description and claims taken in conjunction with the accompanying drawings.

### BRIEF DESCRIPTION OF THE DRAWINGS

This invention, as defined in the claims, can be better understood with reference to the following drawings:

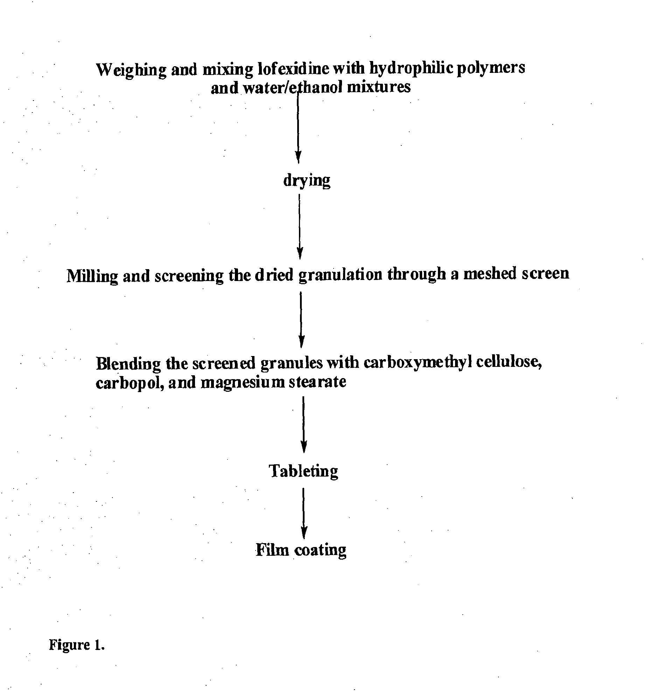

Figure 1 is a flow diagram depicting steps in a wet granulation method for manufacturing the sustained release formulation of the present oral delivery invention comprising lofexidine.

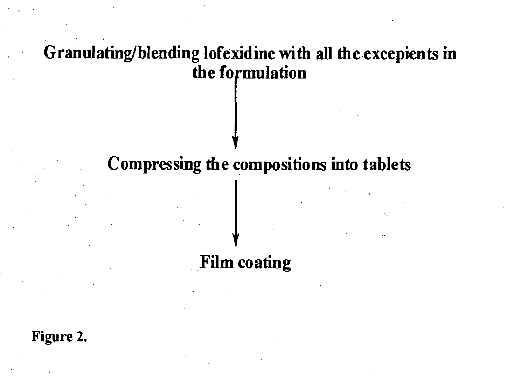

Figure 2 is a flow diagram depicting steps in a dry granulation method for manufacturing the sustained release formulation of the present oral delivery invention comprising lofexidine.

Figure 3 is a flow diagram depicting steps in manufacturing a mixture fast/sustained release formulation of the present oral delivery invention comprising lofexidine.

Figure 4 is a graph demonstrates the *in vitro* release profile of lofexidine from a sustained release oral tablet formulations (Examples 1, 2, and 3)

Figure 5 is a graph demonstrates the *in vitro* release profile of lofexidine from a gel formulation (Example 5) to a donor chamber through porcine skin.

Figure 6 is a graph demonstrates *the in vitro* release profile of lofexidine from a gel formulation (Example 5) to a donor chamber through porcine skin pretreated with microneedles (150 micron)

In the following description of the illustrated embodiments, references are made to the accompanying drawings, which form a part hereof, and in which is shown by way of illustration various embodiments in which the invention may be practiced. It is to be understood that other embodiments may be utilized and structural and functional changes may be made without departing from the scope of the present invention.

### DETAILED DESCRIPTION OF THE INVENTION

Unless defined otherwise, all technical and scientific terms used herein have the same meanings as commonly understood by one of ordinary skill in the art to which this invention belongs. Although any methods and materials similar or equivalent to those described herein can be used in the practice or testing of the present invention, the preferred methods, devices, and materials are now described. All references, publications, patents, patent applications, and commercial materials mentioned herein are incorporated herein by reference for all purposes including for describing and disclosing the methodologies which are reported in the publications which might be used in connection with the invention. Nothing herein is to be construed as an admission that the invention is not entitled to antedate such disclosure by virtue of prior invention.

In order to provide a clear and consistent understanding of the specification and claims, including the scope to be given such terms, the following definitions are provided:

The term "administration" defined herein includes oral and transdermal application of the pharmaceutically active compounds and the pharmaceutical compositions.

By "compatible" herein is meant that the components of the compositions which comprise the present invention are capable of being mixed without interacting in a manner which would substantially decrease the efficacy of the pharmaceutically active compound under ordinary use conditions.

The terms "effective amount" or "pharmaceutically effective amount" refer to a sufficient amount of the agent to provide the desired biological result, which is substantially nontoxic. That result can be reduction and/or alleviation of the signs, symptoms, or causes of a disease, such as the treatment of opiate addicts, migraine, neuropathic pain, and other therapeutic indications related to lofexidine. An appropriate "effective" amount in any individual case may be determined by one of ordinary skill in the art using routine experimentation.

As used herein, the term "excipient" means the substances used to formulate active pharmaceutical ingredients (API) into pharmaceutical formulations; in a preferred embodiment, an excipient does not lower or interfere with the primary therapeutic effect of the API. Preferably, an excipient is therapeutically inert. The term "excipient" encompasses carriers, diluents, vehicles, solubilizers, stabilizers, bulking agents, acidic or basic pH-adjusting agents and binders. Excipients can also be those substances present in a pharmaceutical formulation as an indirect or unintended result of the manufacturing process. Preferably, excipients are approved for or considered to be safe for human and animal administration, *i.e.,* GRAS substances (generally regarded as safe). GRAS substances are listed by the Food and Drug administration in the Code of Federal Regulations (CFR) at 21 CFR 182 and 21 CFR 184, incorporated herein by reference.

As used herein, the term "formulate" refers to the preparation of a drug, e.g., lofexidine, in a form suitable for administration to a mammalian patient, preferably a human. Thus, "formulation" can include the addition of pharmaceutically acceptable excipients.

The term "permeation enhancer" or "penetration enhancer" as used herein refers to an agent that improves the rate of transport of a pharmacologically active agent (e.g., lofexidine) across the transdermal tissues. Typically a penetration enhancer increases the permeability of skin to a pharmacologically active agent. Penetration enhancers, for example, enhance the rate at which the pharmacologically active agent permeates through membranes and enters the bloodstream. The ability of a penetration enhancer to enhance permeation can be observed, for example, by measuring the flux of the pharmacologically active agent across animal or human membranes as described in the Examples herein below. An "effective" amount of a permeation enhancer as used herein means an amount that will provide a desired enhancement in skin permeability to provide, for example, the desired depth of penetration of a selected compound, rate of administration of the compound, and amount of compound delivered.

By "pharmaceutically acceptable" or "pharmacologically acceptable" is meant a material which is not biologically or otherwise undesirable, i.e., the material may be administered to an individual without causing any undesirable biological effects or interacting in a deleterious manner with any of the components of the composition in which it is contained.

As used herein, a "pharmaceutically acceptable carrier" is a material that is nontoxic and generally inert and does not affect the functionality of the active ingredients adversely. Examples of pharmaceutically acceptable carriers are well known and they are sometimes referred to as diluents, vehicles or excipients. The carriers may be organic or inorganic in nature. In addition, the formulation may contain additives such as coloring agents, thickening or gelling agents, emulsifiers, wetting agents, buffers, stabilizers, and preservatives such as antioxidants.

The term "pharmaceutical composition" as used herein shall mean a composition that is made under conditions such that it is suitable for administration to humans, e.g., it is made under current good manufacturing practice (cGMP) conditions and contains pharmaceutically acceptable excipients, e.g., without limitation, stabilizers, pH adjusting agents, bulking agents, buffers, carriers, diluents, vehicles, solubilizers, and binders.

As used herein, the term "subject" encompasses mammals and non-mammals. Examples of mammals include, but are not limited to, any member of the Mammalian class: humans, non-human primates such as chimpanzees, and other apes and monkey species; farm animals such as cattle, horses, sheep, goats, swine; domestic animals such as rabbits, dogs, and cats; laboratory animals including rodents, such as rats, mice and guinea pigs, and the like. Examples of non-mammals include, but are not limited to, birds, fish and the like. The term does not denote a particular age or sex.

As used herein, the terms "treating" or "treatment" of a disease include preventing the disease, *i. e.* preventing clinical symptoms of the disease in a subject that may be exposed to, or predisposed to, the disease, but does not yet experience or display symptoms of the disease; inhibiting the disease, i.e., arresting the development of the disease or its clinical symptoms, such as by suppressing or relieving the disease, i.e., causing regression of the disease or its clinical symptoms.

**General**

This invention relates to sustained release oral and transdermal pharmaceutical formulations and delivery systems comprising lofexidine.

More specifically, the invention features compositions and methods for sustained release and combination sustained release/rapid release oral and transdermal formulations comprising lofexidine formulated in capsules, oral tablets, transdermal formulations, or a patch-needle hybrid (Microneedle) to deliver lofexidine by itself or in combination with other medications for many therapeutic uses, including but not restricted to: opiate detoxification, alcohol withdrawal syndrome, decrease stress-induced reinstatement of seeking addictive materials, pain management such as neuropathic pain and migraine, alleviate tobacco and alcohol withdrawal symptoms treat intraocular pressure (IOP), anti-diarrheal agent, treat cardiovascular complications in patients with obstructive sleep apnea, and to prevent adverse effects of N-methyl-D-aspartate (NMDA) antagonists or schizophrenia-associated (NMDA) receptor hypofunction, and other therapeutic indications related to lofexidine.

The present invention further includes methods for administering a composition of the present invention to a subject in need thereof. Compositions of the present invention comprising lofexidine can be employed, for example, for the treatment of a variety of conditions and/or disease states which have been historically treated by oral doses of lofexidine.

More particularly, the present invention concerns the transdermal and sustained release oral administration of lofexidine. "Lofexidine" refers to the compound: 2-[1-(2,6 dichlorophenoxy)ethyl]-4,5-dihydro- 1H-Imidazole, and has the following formula:

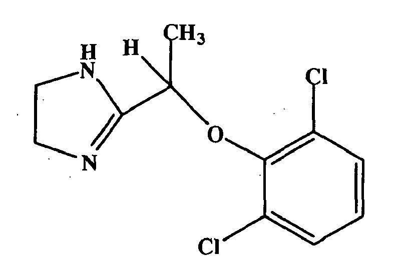

In the present invention, lofexidine can exist in a free base form or as any pharmaceutically acceptable salt. Pharmaceutically acceptable salt refers to pharmaceutically acceptable salts of lofexidine which are derived from a variety of organic and inorganic counter ions that are well known in the art and include, by way of example only, hydrochloride, hydrobromide, tartrate, mesylate, acetate, maleate, oxalate and the like.

For the purposes of the present invention, lofexidine hydrochloride is preferred; however, other pharmacologically acceptable moieties thereof can be utilized as well.

The term "lofexidine" as used herein includes the free base form of this compound as well as pharmacologically acceptable acid addition salts thereof.

The lofexidine and lofexidine salts for use according to the invention may be in the form of a free amine (i.e. -NH-) or more preferably in the form of a pharmaceutically acceptable salt. In one embodiment, the salts are acid addition salts with physiologically acceptable organic or inorganic acids. Suitable acids include, for example, hydrochloric, hydrobromic, phosphoric, sulphuric and sulphonic acids. In another embodiment, the salts are acid addition salts with hydrochloric acid. Procedures for salt formation are conventional in the art.

In one embodiment, the lofexidine for use in the invention is an enantiomerically pure (e.g. it has an enantiomeric excess of at least 90 %, in another embodiment at least 95 %, in yet another embodiment at least 99 % by weight). In one embodiment, the lofexidine enantiomer for use in the invention is (-)-lofexidine. In another embodiment, the pharmaceutically acceptable salts of lofexidine are those formed from (-)-lofexidine, (+)-lofexidine, or a racemic mixture (-/+)-lofexidine. Enantiomerically pure lofexidine and pharmaceutically acceptable salts thereof may be prepared by conventional procedures described in the art (e.g. as described in J. Med. Chem., 1986, 29, 991183-1188).

The lofexidine therapeutic effect can be achieved to a degree sufficient to cause a relief of opiate addiction symptoms, migraine or treatment of neuropathic pain by the sustained release delivery of lofexidine through transdermal and sustained release oral delivery so as to maintain an adequate plasma concentration of lofexidine. The amount of lofexidine administered is an amount sufficient to cause therapeutic effect but is low enough not to cause substantial intolerable adverse side effects. As used herein, "substantial intolerable adverse side effects" include those effects caused by either the delivery system or the alpha-two receptor agonist which are incompatible with the health of the user or which are so unpleasant as to discourage the continued use of the composition. Such effects include, for example, hypotension, nausea, vomiting, impaired vision, and diaphoresis.

In one embodiment, a "detoxifying amount of lofexidine" includes an effective amount of lofexidine which may substantially saturate, bind to, or block an effective number of the opioid receptors in a subject. The terms "substantially saturate" and "substantially block" an effective number of opioid receptors include about 75%, about 80%, about 85%, about 90%, about 95%, or higher, saturation or blockage of the opioid receptors in a subject.

In one aspect, a detoxifying amount comprises about 0.1 mg to about 10 mg of lofexidine. The dosage of lofexidine may be about 1 mg to about 8 mg, or about 2 mg to about 6 mg, or about 3 mg to about 5 mg, or about 1 mg, 2 mg, 3 mg, 4 mg, 5 mg, 6 mg, 7 mg 8 mg, 9 mg or 10 mg of lofexidine.

The term "opioid" refers to natural, synthetic, or semi-synthetic compounds or compositions including metabolites of such compounds or compositions which bind to specific opioid receptors and have agonist (activation) or antagonist (inactivation) effects at these receptors, such as opioid alkaloids, including the agonist morphine and its metabolite morphine-6-glucuronide and the antagonist naltrexone and its metabolite and opioid peptides, including enkephalins, dynorphins and endorphins. The opioid can be present as a member selected from an opioid base and an opioid pharmaceutically acceptable salt. The pharmaceutically acceptable salt embraces an inorganic or an organic salt. Representative salts include hydrobromide, hydrochloride, mucate, succinate, n-oxide, sulfate, malonate, acetate, phosphate dibasic, phosphate monobasic, acetate trihydrate, bi(heplafluorobutyrate), maleate, bi(methylcarbamate), bi(pentafluoropropionate), mesylate, bi(pyridine-3-carboxylate), bi(trifluoroacetate), bitartrate, chlorhydrate, fumarate and sulfate pentahydrate. The term "opiate" refers to drugs derived from opium or related analogs.

A first aspect of the invention provides a method for relieving symptoms associated with illness or associated with the treatment of illness in a mammalian subject, comprising lofexidine mixed with a polymer blend which consists of at least one pharmaceutically acceptable hydrophobic rate controlling material, at least one pharmaceutically acceptable hydrophilic rate controlling material, at least one pharmaceutically acceptable rate controlling polymer such as a pharmaceutically acceptable amphipathic polymer, at least one pharmaceutically acceptable non-polymer rate controlling material, or any combinations thereof for oral sustained release delivery.

A second aspect of the invention provides a method for relieving symptoms associated with illness or associated with the treatment of illness in a mammalian subject, comprising lofexidine, selecting at least one permeation enhancer from the group consisting of propylene glycol monolaurate, diethylene glycol monoethyl ether, an oleoyl macrogolglyceride, a caprylocaproyl macrogolglyceride, and an oleyl alcohol, and delivering lofexidine and the permeation enhancer transdermally to treat an illness.

A third aspect of the invention provides an occlusive body for the delivery of lofexidine, comprising an impermeable backing, a rate-controlling microporous membrane, said backing and membrane defining a cavity there between, a quantity of lofexidine disposed within the cavity, a permeation enhancer disposed within the cavity, and a viscous flowable gel confined within the cavity for immobilizing the lofexidine and the permeation enhancer.

A fourth aspect of the invention provides a combination sustained release/rapid release pharmaceutical oral and transdermal formulation. More specifically it provides a method for increasing and enhancing the transdermal delivery of lofexidine in a subject, comprising contacting the subject's skin with a microneedle. Preferably, the lofexidine is administered by creating a microneedle-treated site in the skin of a subject by inserting microneedles, followed by applying the lofexidine to the microneedle-treated site.

In a preferred embodiment, a sustained release formulation comprises about 0.1 to about 50 weight % lofexidine. In another preferred embodiment a modified release formulation comprises about 1 to about 10 weight % lofexidine

In a preferred embodiment, a sustained release transdermal formulation comprises also: about 1 to about 50 weight % water; about 10 to about 98 weight % propylene glycol; and about 1 to about 10 weight % hydroxypropylethylcellulose polymer. In another preferred embodiment a modified release transdermal formulation comprises, in addition to lofexidine, about 5 to about 30 weight % water; about 30 to about 70 weight % propylene glycol; about 1 to about 5 weight % hydroxypropylethylcellulose polymer; and about 0.01 to 5% preservative.

In a preferred embodiment, a sustained release oral formulation comprises also about 10 to about 90% binder; about 1 to about 50% hydrophilic polymer, about 0.1 to about 50% hydrophobic polymer, about 0.1 to about 2% lubricant and glidant, and about 0.1 to about 1% colorant. In another preferred embodiment, a sustained release oral formulation comprises, in addition to lofexidine, about 30 to about 50% lactose; about 1 to 2.5% hydroxypropylmethylcellulose, approximately 2 to about 50 % acrylic resin, approximately 0.5 to about 1% magnesium stearate, and approximately 0.1 to about 0.5% colorant.

Examples of hydrophobic polymers and hydrophilic polymers that are pharmaceutically acceptable include, but are not limited to, certain pharmaceutically acceptable acrylic resins, pharmaceutically acceptable acrylic polymers, pharmaceutically acceptable cellulose ethers and pharmaceutically acceptable biologically derived materials. Examples of pharmaceutically acceptable acrylic resins and pharmaceutically acceptable acrylic polymers include, but not limited to, acrylic acid and alkylacrylic acid copolymers (which are also anionic polymers), methyl methacrylate copolymers, ethoxyethyl methacrylates, cyanoethyl methacrylate, poly(acrylic acid), poly(methacrylic acid), methacrylic acid alkylamide copolymer, poly(methyl methacrylate), polymethacrylate, poly(methyl methacrylate) copolymer, polyacrylamide, aminoalkyl methacrylate copolymer, poly(methacrylic acid anhydride), and glycidyl methacrylate copolymers, methacrylic acid ester copolymers, polyvinyl acetate phthalate (PVAP), polyvinyl acetate and polyvinyl pyrrolidone and polyvinyl alcohol (PVA). Examples of pharmaceutically acceptable cellulose ethers include, but not limited to, hydroxyalkylcelluloses and carboxyalkylcelluloses, such as, hydroxypropylcellulose (HPC), hydroxypropylmethylcellulose (HPMC), hydroxyethylcellulose (HEC), hydroxypropylethylcellulose (HPEC), methyl cellulose (MC), ethyl cellulose (EC), cellulose acetate (CA), cellulose acetate butyrate, cellulose acetate propionate, hydroxypropylmethylcellulose phthalate (HPMCP) (which is also an anionic polymer), carboxyl methylcellulose (CMC), cellulose acetate phthalate (CAP) (which is also an anionic polymer). Examples of pharmaceutically acceptable biologically derived materials include, but are not limited to, polysaccharides or their derivatives, such as, but not limited to, gums (such as, xantham gum, locust bean gum), sodium alginate, shellac, zein, and the like. In one embodiment of a sustained release formulation, the hydrophilic polymer is hydroxypropylethylcellulose.

Water-insoluble polymers which are suitable for use in the sustained release formulation are polymers which generally do not dissolve in solutions of a pH below 5, and dissolve more slowly in basic solutions than the hydrophilic polymer. Because the polymer is insoluble in low pH environments such as those found in gastric fluid, it aids in retarding drug release in those regions. Likewise, because the polymer dissolves more slowly in solutions of higher pH than hydrophilic polymers, it aids in retarding drug release throughout the intestines. This overall delayed release results in a more uniform serum concentration of lofexidine. In a preferred embodiment, a sustained release formulation comprises the acrylic resin CARBOPOL 974P.

The rate controlling materials can be any suitable weight percentage of the pharmaceutical composition, and preferably make up between 1 % (w/w) to about 90% (w/w) of the pharmaceutical composition. In addition to hydrophilic polymers and hydrophobic polymers, the rate controlling material can comprise a pharmaceutically acceptable non-polymer material. Examples of pharmaceutically acceptable non-polymer rate controlling materials include, but are not limited to, certain pharmaceutically acceptable long chain substituted or un-substituted hydrocarbons. Examples of pharmaceutically acceptable long chain substituted or unsubstituted hydrocarbons include, but are not limited to, fatty acid esters, fatty acid glycerides (mono-, di-, and tri-glycerides), stearic acid, glyceryl monostearate, glyceryl behenate, lauryl, myristyl, stearyl, cetyl or cetostearyl alcohol, polyethylene glocol, poly(ethylene oxide) and natural and synthetic waxes (such as, but not limited to, beeswax, glycowax, castor wax and camauba wax).

A sustained release formulation of the present invention may further comprise pharmaceutical additives including, but not limited to:
lubricants such as magnesium stearate, calcium stearate, zinc stearate,
powdered stearic acid, hydrogenated vegetable oils, talc, polyethylene glycol, and mineral oil; colorants such as Emerald Green Lake and
various FD&C colors; binders such as sucrose, lactose, starch paste, acacia, tragacanth, povidone polyethylene glycol, Pullulan and corn syrup;
glidants such as colloidal silicon dioxide and talc; surface active agents such as sodium lauryl sulfate, dioctyl sodium sulfosuccinate, triethanolamine, polyoxyetiylene sorbitan, poloxalkol, and quarternary ammonium salts; preservatives and stabilizers; excipients such as lactose, mannitol, glucose, fructose, xylose, galactose, sucrose, maltose, xylitol, sorbitol, chloride, sulfate and phosphate salts of potassium, sodium, and
magnesium; and/or any other pharmaceutical additives known to those of skill in the art. In one preferred embodiment, a sustained release formulation further comprises magnesium stearate.

A sustained release formulation of the present invention comprises lofexidine, at least one hydrophilic polymer, at least one water-insoluble polymer, and at least one pharmaceutical additive in any appropriate percent quantity which permits dissolution of drug ingredients that result in a therapeutically effective serum concentration profile.

In one embodiment of the invention, the delivery vehicle is for topical administration to the skin and includes but is not limited to a transdermal device, a cream, a lotion or an ointment which delivers a pharmacologically effective amount of lofexidine to the subject in need. In another embodiment of the invention the delivery vehicle is a transdermal device occlusive body (i.e., a transdermal delivery patch)

The transdermal device for the transdermal delivery of an effective amount of lofexidine comprises: a backing layer, a reservoir layer for the lofexidine or any pharmaceutically acceptable salt of lofexidine, a pharmaceutically effective carrier, optionally a control membrane or non controlling microporous membrane, optionally an adhesive, and optionally a protective peel strip.

In the preferred embodiment of the present invention, the drug reservoir layer contains lofexidine. The excipient is chosen appropriately with its component comprising: drug component carrier, surfactant, filler, and polymer matrix. The suitable drug component carrier is selected from a common group comprising: light mineral oil, myristates, isostearates, glycerides, polyethylene glycol and the derivative thereof, and the mixture thereof. The suitable surfactant comprises: vitamin E and the derivative thereof, oleic acid and the derivative thereof, and the mixture thereof. The suitable filler is a commonly available one, such as silicone dioxide. The polymer matrix serves as the adhesive, hence the common use comprises:
Acry series adhesive polymer and polyisobutylene polymer.

Skin permeability can be increased through the use of chemical enhancers, electrical enhancers via electroporation or iontophoresis, ultrasonic enhancers, and a variety of other approaches. However, delivering macromolecules into the skin remains a significant challenge. See, e.g., Park et al., J. Controlled Release 104 (2005) 51-66 and Martanto et al., Pharm. Res. 21 (2004).

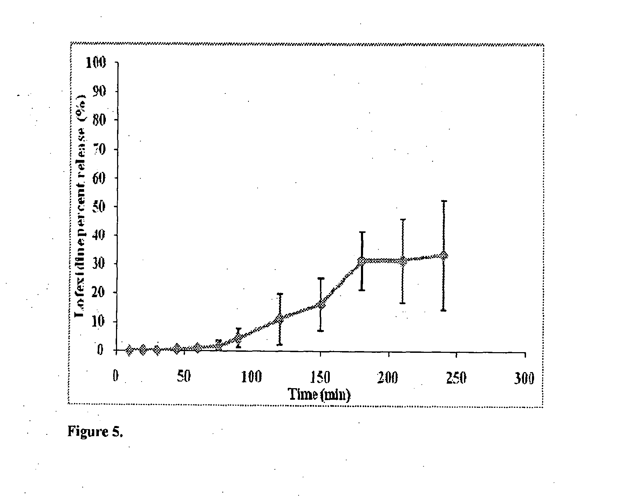

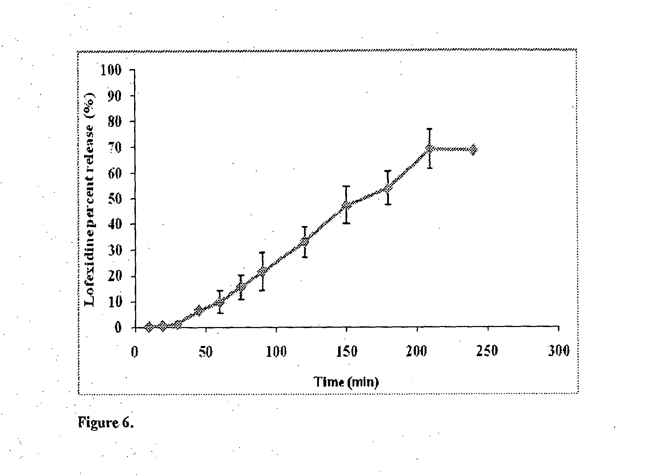

An alternative approach to increase transdermal transport involves using arrays of microscopic needles (or "microneedles ") to pierce the skin, thus creating micrometer-scale transport pathways. Microneedles provide a minimally invasive means to transport molecules into the skin, as the channels they create are extremely small on a clinical level. However, because the channels are much larger than macromolecules, such channels dramatically increase skin permeability, see Figures 5 and 6.

Currently, microneedles are made from silicon, biodegradable polymers, and stainless steel. Microneedles can be solid or hollow. Solid microneedles can be used to create holes in the skin, followed by application of a transdermal patch to the skin surface. Alternatively, solid microneedles can be first coated with a drug and then inserted into the skin. Hollow microneedles can also be used, to facilitate active fluid flow through the needle bore and into the skin. See, e.g., Prausnitz, Adv. Drug. Deliv. Rev. 56 (2004) 581-587, for a review.

In one embodiment, the microneedles delivery systems consist of short, micrometer-scale needles that can be used for drug delivery, allowing a drug to diffuse to the rich capillary bed of the dermis for uptake and subsequent systemic distribution in the blood stream. Since these needles would be inserted no deeper than the outmost, non-innervated layer of the skin, this technique would allow painless delivery. A microneedle delivery system achieves the advantages of subcutaneous drug delivery in a non attention drawing and minimally invasive manner. Since studies report needle size and fear of pain as two major reasons for injection anxiety, such a device could improve patient acceptance, and the development of a "controlled release"-design could further prevent long-term complications.

In one embodiment, the lofexidine is administered by creating a microneedle-treated site in the skin of a subject by inserting microneedles, followed by applying the lofexidine to the microneedle-treated site.

In another embodiment, the composition further comprises a permeation enhancer (penetration enhancer). Permeation enhancers include, but are not limited to, sulfoxides, surfactants, 1-substituted azacycloheptan-2-ones, fatty alcohols, fatty acids, fatty acid esters, polyols and esters thereof, alkanones and organic acids. In another embodiment of the invention, these permeation enhancers include, but are not limited to, sulfoxides such as dimethylsulfoxide and decylmethylsulfoxide; surfactants such as sodium laurate, sodium lauryl sulfate, cetyltrimethylammonium bromide, benzalkonium chloride, poloxamer (231, 182, 184), tween (20, 40, 60, 80) and lecithin; the 1-substituted azacycloheptan-2-ones, particularly 1-n-dodecylcyclazacycloheptan-2-one; fatty alcohols such as lauryl alcohol, myristyl alcohol, oleyl alcohol and the like; fatty acids such as lauric acid, oleic acid and valeric acid; fatty acid esters such as isopropyl myristate, isopropyl palmitate, methylpropionate, and ethyl oleate; polyols and esters thereof such as propylene glycol, ethylene glycol, glycerol, butanediol, polyethylene glycol, and polyethylene glycol monolaurate, amides and other nitrogenous compounds such as urea, dimethylacetamide (DMA), dimethylformamide (DMF), 2-pyrrolidone, 1-methyl-2-pyrrolidone, ethanolamine, diethanolamine and triethanolamine, terpenes; alkanones, and organic acids, particularly salicylic acid and salicylates, citric acid and succinic acid. As noted earlier herein, "Percutaneous Penetration Enhancers", eds. Smith et al. (CRC Press, 1995), which is incorporated herein by reference thereto, provides an excellent overview of the field and further information concerning possible secondary enhancers for use in conjunction with the present invention. More permeation enhancer(s) suitable to be used with the present invention may be known by those skilled in the art.

In one embodiment, the permeation enhancer is present in an amount of from about 0.1 to about 30% w/w depending on the type of compound. In one embodiment, the permeation enhancers are fatty alcohols and fatty acids. In another embodiment, the permeation enhancers are fatty alcohols. In one embodiment, the fatty alcohols have the formula the CH₃(CH₂)ₙ(CH)ₘCH₂OH wherein n ranges from (8-m) to (16-m) and m=0-2. In one embodiment, the concentration range of the penetration enhancer(s) is, depending on the type of permeation enhancer, a concentration of between about 0.1 and about 10 weight percent, as known by one skilled in the art. In one embodiment, the penetration enhancer comprises myristyl alcohol in a concentration of between about 0.1 and about 2 weight percent.

In some embodiments, the permeation enhancer is chosen from: a bile salt, sodium dodecyl sulfate, dimethyl sulfoxide, sodium lauryl sulfate, a derivative of a saturated or unsaturated fatty acid, a surfactant, a bile salt analog, and a derivative of a bile salt. In some embodiments the permeation enhancer is a synthetic permeation enhancer.

In another embodiment, the composition comprises antioxidant(s), for example, tocopherol and derivatives, ascorbic acid and derivatives, butylated hydroxyanisole, butylated hydroxytoluene, fumaric acid, malic acid, propyl gallate, sodium metabisulfite and derivatives, is a concentration of about 0.01 to about 5 weight percent; more preferred is a concentration of about 0.1 to about 0.5 weight percent, depending on the type of antioxidant used, as known by one skilled in the art.

In another embodiment, the composition comprises buffering agent(s), for example, carbonate buffers, citrate buffers, phosphate buffers, acetate buffers, hydrochloric acid, lactic acid, tartaric acid, inorganic and organic bases, is a concentration of about 1 to about 10 weight percent, another embodiment is a concentration of about 2 to about 5 weight percent, depending on the type of buffering agent(s) used, as known by the one skilled in the art. In one embodiment, the concentration range of said buffering agents are those compositions having a pH close to the physiologic pH of the mucosal membranes, between about pH 2.0 and about pH 10.0, and in another embodiment is between about pH 3.0 and pH 7.0. Concentrations of the buffering agent(s) may vary, however, as known by the one skilled in the art. The buffering agent may replace up to 100% of the water amount within the composition.

In another embodiment, the composition comprises preservatives such as benzalkonium chloride and derivatives, benzoic acid, benzyl alcohol and derivatives, bronopol, parabens, centrimide, chlorhexidine, cresol and derivatives, imidurea, phenol, phenoxyethanol, phenylethyl alcohol, phenylmercuric salts, thimerosal, sorbic acid and derivatives. The preservative is present from about 0.01 to about 10% w/w depending on the type of compound used, as known by the one skilled in the art.

As discussed previously herein, this invention relates also to a combination sustained release/rapid release oral and transdermal pharmaceutical formulations and delivery systems comprising lofexidine.

The sustained release formulations of the present invention may be manufactured according to any appropriate method known to those of skill in the art of pharmaceutical manufacture.

The backing layer, reservoir layer, control membrane, adhesive and protective peel strip can be formed using conventional teaching in the art such as those referred to in U.S. Patent 6,818,226 (Dermal penetration enhancers and drug delivery systems involving same); U.S. Patent 6,791,003 (Dual adhesive transdermal drug delivery system); U.S. Patent 5,858,393 (Transdermal formulation); U.S. Patent 5,612,382 (Composition for percutaneous absorption of pharmaceutically active ingredients); U.S. Patent 5,464,387 (Transdermal delivery device); U.S. Patent 5,023,085 (Transdermal flux enhancers in combination with iontophoresis in topical administration of pharmaceuticals; U.S. Patent 4,654,209 (Preparation of percutaneous administration), each of which is incorporated by reference.

The sustained release dosage form of the present invention may be manufactured by standard techniques known by those skilled in the art. For example, the oral dosage form can be manufactured using a wet granulation technique. In the wet granulation technique, a drug and the ingredients comprising the drug composition are mixed in a mixer to form a drug blend. Next, other ingredients comprising the drug composition can be optionally dissolved in a portion of the granulation fluid to form a wet blend. The granulation fluid can be in the form of an aqueous solution or can contain one or more solvents. Then, the prepared wet blend is slowly added to the drug blend with continual mixing in the mixer. The granulating fluid is added until wet granules are produced. The wet granules are then optionally forced through a predetermined screen onto oven trays. The blend is dried under suitable conditions, for example, 12 to 24 hours at 24°C to 55°C in a forced-air oven. The dried granules are then sized. Next, a suitable lubricant such as magnesium stearate is added to the drug granulation. The powder blend can then be compressed into tablets using a rotary press or a hydraulic press. The speed of the press can be set at 20 rpm and the maximum load set can be set at 2 tons.

Similarly, thermal forming, melt granulation, dry granulation, direct compression, and various other suitable methods and techniques known in the art can be used to make the sustained release oral dosage forms of the present invention.

The oral sustained-release formulations can be manufactured by dissolving lofexidine and adhesives in ethanol, mixing with sustained-release adjuvants and fillers, wetting with ethanol, granulating, drying. The granules can be filled in capsules (i.e. hard gelatin capsules) or could be processed for tablet making and coating. The adhesive material could be povidone K30 or any pharmaceutically suitable material. The lubricant could be magnesium stearate or any pharmaceutically acceptable material. Optionally, the glidant could be silicon dioxide or any pharmaceutically acceptable material that may be added into sustained-release formulation. The filler could be microcrystalline cellulose, lactose or any pharmaceutically acceptable material. The adjuvants could be used as one of each type or in mixed combination from the same class to optimize the sustained-release kinetics of the formulation. A description of representative sustained release carrier materials and adjuvants can be found in the Remington: The Science and Practice of Pharmacy (20th edition, Lippincott Williams & Wilkens Publishers (2003)), which is incorporated herein by reference in its entirety.

Dosage forms described herein may be formulated to comprise various excipients, binders, carriers, disintegrants, coatings, etc. Pharmaceutical preparations can be obtained by mixing one or more solid excipients with a pharmaceutical composition as described herein, optionally grinding the resulting mixture, and processing the mixture of granules, after adding suitable auxiliaries, if desired, to obtain sustained release pharmaceutical compositions suitable for use in various forms, e.g., as pills, tablets, powders, granules, capsules, liquids, sprays, gels, syrups, slurries, suspensions and the like, in bulk or unit dosage forms, for oral ingestion by a patient to be treated.

The pharmaceutical compositions can additionally include preservatives, solubilizers, stabilizers, wetting agents, emulsifiers, sweeteners, colorants, flavorings, buffers, coating agents, or antioxidants. Dissolution or suspension of the active ingredient in a vehicle such as water or naturally occurring vegetable oil like sesame, peanut, or cottonseed oil or a synthetic fatty vehicle like ethyl oleate or the like may be desired. Buffers, preservatives, antioxidants and the like can be incorporated according to accepted pharmaceutical practice. The compound can also be made in microencapsulated form. Absorption enhancing preparations, for example, liposomes, nanoparticles, can also be utilized. Those skilled in the art can formulate sustained-release dosage forms containing one or more of the foregoing ingredients by routine experimentation.

Furthermore, the oral sustained release formulations could be manufactured as drug delivery systems including at least one population of beads, where each population of beads includes lofexidine alone or in combination with other pharmaceutically active ingredients. The beads may be selected from immediate-release beads, enteric-release beads, and/or sustained-release beads. The dosage forms of the present invention will be useful for treating conditions including therapeutic use of lofexidine alone or in combination with other pharmaceutically active compound to treat multi-disease conditions.

The sustained release formulation can be engineered in combination with an immediate release formulation to deliver a therapeutically active loading dose.

The sustained release oral and transdermal dosage forms of the present invention can be administered to a human patient in a manner to provide effective concentrations of lofexidine to quickly combat existing symptoms (e.g., within about 1 hour) and provide a sustained release to maintain levels of lofexidine sufficient to exert the therapeutic effect for up to about 24 hours after oral administration and up to about 7 days after transdermal administration. In a preferred embodiment, the sustained release formulations and delivery systems provide a sustained release to maintain levels of lofexidine sufficient to exert the therapeutic effect for up to about 12 hours after oral administration and up to about 3 days after transdermal administration.

In one embodiment, the invention is directed to a pharmaceutical composition comprising from about 0.01 mg to about 10 mg of lofexidine. In some embodiments, the pharmaceutical composition comprises from about 0.1 mg to about 4 mg of lofexidine.

In another aspect, the present invention includes methods for administering lofexidine to a human subject in need thereof. For example, the method may comprise providing a composition of the present invention for transdermal delivery of lofexidine. Furthermore, the present invention provides compositions and methods for oral sustained release delivery of lofexidine.

The methods of manufacturing of the present invention may include dispensing compositions of the present invention into appropriate containers. The compositions of the present invention may be packaged, for example, in unit dose or multi -dose containers. The container typically defines an inner surface that contains the composition. Any suitable container may be used. The inner surface of the container may further comprise a liner or be treated to protect the container surface and/or to protect the composition from adverse affects that may arise from the composition being in contact with the inner surface of the container. Exemplary liners or coating materials include, but are not limited to high density polyethylene, low density polyethylene, very low density polyethylene, polyethylene copolymers, thermoplastic elastomers, silicon elastomers, polyurethane, polypropylene, polyethylene terephthalate, nylon, flexible polyvinylchloride, natural rubber, synthetic rubber, and combinations thereof. Liners or coating material are typically substantially impermeable to the composition and typically to the individual components of the composition.

A number of types of containers are known in the art, for example, packets with ruptureable barriers (see, for example, U.S. Pat. Nos. 3,913,789, 4,759,472, 4,872,556, 4,890,744, 5,131,760, and 6,379,069), single-use packets (see, for example, U.S. Pat. Nos. 6,228,375, and 6,360,916), tortuous path seals (see, for example, U.S. Pat. Nos. 2,707,581, 4,491,245, 5,018,646, and 5,839,609), and various sealing valves (see, for example, U.S. Pat. Nos. 3,184,121, 3,278,085, 3,635,376, 4,328,912, 5,529,224, and 6,244,468). One example of a unit dose container is a flexible, foil packet with a polyethylene liner.

In one embodiment, the pharmaceutical compositions of the present invention further comprise an effective amount of at least one opioid. In another embodiment, the opioid is selected from the group consisting of opium, morphine, heroin, pethidine, methadone, buprenorphine, butorphanol, codeine, fentanyl, hydrocodone, hydromorphone, levorphanol, meperidine, oxycodone, pentazocine, propoxyphene, or tramadol, pharmaceutical formulations, pharmaceutical salts, or mixtures or combinations there of.

In one embodiment, the pharmaceutical compositions of the present invention further comprise an effective amount of at least one opioid antagonist. In another embodiment, the opioid antagonist is selected from the group consisting of 7-benzylidenenaltrexone, beta-funaltrexamine, buprenorphine, butorphanol, chlornaltrexamine, clocinnamox, connective tissue-activating peptide, cyclazocine, diprenorphine, ICI 154129, levallorphan, meptazinol, methylnaltrexone, N,N-diallyl-tyrosyl-alpha-aminoisobutyric acid-phenylalanyl-leucine, nalbuphine, nalmefene, nalorphine, naloxone, naltrexone, or naltrindole, or mixtures or combinations thereof.

In one embodiment, the pharmaceutical composition of the present invention further comprise an effective amount of at least one sedative, hypnotic, anxiolytic, or antihistamine. In another embodiment, the pharmaceutical composition of the present invention further comprise an effective amount of at least one sedative or hypnotic, such as the barbiturates, amylobarbitone, butobarbitone and pentobarbitone and other hypnotics and sedatives such as choral hydrate, chlormethiazole, hydroxyzine and meprobamatc; anxiolytic, such as tybamate, tetrazepam, meprobamate, chlormezanone; or antihistamine, such as meclozine, cyclizine, chiorcyclizine, hydroxyzine, brompheniramine, chlorpheniramine, clemastine, cyproheptadine, dexchlorpheniramine, diphenhydramine, diphenyle, doxylamine, mebhydrolin, mepyramine, pheniramine, orphenadrine, tripolidine, azatadine, diphenylpyraline, methdilazine, terfexine, astemizole, loratidine and cetirizine.

In one embodiment, the pharmaceutical composition of the present invention further comprises an effective amount of at least one muscle relaxant. In another embodiment, the pharmaceutical composition of the present invention further comprise an effective amount of at least one muscle relaxant, such as alcuronium, atracurium, baclofen, carisoprodol, quinine derivatives, chlormezanone, chlorphenesin, chlorzoxazone, cyclobenzaprine hydrochloride, dantrolene, decamethonium bromide, diazepam, dimethyltubocurarinium, phenyramidol, gallamine triethiodide, guaifenesin, hexafluronium, mephenesin, metaxalone, methocarbamol, orphenadrine, phenprobamate, succinylcholine, tetrazepam, tizanidine, tubocurarinchloride.

In one embodiment, the pharmaceutical compositions of the present invention further comprise an effective amount of at least one cannabinoid agonist, such as marinol.

In one embodiment, the present invention provides for a method for treating an opiate addiction in a subject comprising administering to a subject with an opiate addiction, an effective amount of a lofexidine transdermally or through oral sustained release formulations on a treatment day. The method may further comprise administering to the subject an effective amount of an opiate on at least one treatment day. The method may further comprise administering to the subject an effective amount of a sedative on at least one treatment day. In one embodiment, the number of days of treatment range from about 2 days to about 20 days.

Lofexidine can also be brought into a viscous basis via systems conventionally used, for example, natural gums, methylcellulose and derivatives, acrylic polymers (carbopol) and vinyl polymers (polyvinylpyrrolidone).

In the present compositions, many other excipients known in the art can be added such as preservatives, surfactants, co-solvents, adhesives, antioxidants, buffers, viscosity enhancing agents and agents to adjust the pH and the osmolarity.

In one embodiment, the formulation may be presented as capsules, tablets, caplets, pills, powders, granules or a suspension suitable for sustained release oral delivery, prepared by conventional means with pharmaceutically acceptable excipients, e.g., with conventional additives or fillers and binders such as lactose, mannitol, corn starch or potato starch; with binders or binding agents such as crystalline cellulose, cellulose derivatives, acacia, corn starch (including pregelatinized) or gelatins; with disintegrators or disintegrants such as corn starch, potato starch or sodium carboxymethyl-cellulose; or with lubricants or wetting agents such as talc or magnesium stearate. Tablets may be coated, including by methods well known in the art. The formulation may be presented as an immediate-release or as a slow-release, sustained-release or controlled-release form. The formulation may also be presented as a solid drug matrix, for example, on a handle. In another embodiment, the formulation may be presented as liquid preparations for oral administration may take the form of, for example, solutions, syrups or suspensions, or they may be presented as a dry product for constitution with water or other suitable vehicle before use. Such liquid preparations may be prepared by conventional means with pharmaceutically acceptable additives such as suspending agents (e.g., sorbitol syrup, methyl cellulose or hydrogenated edible fats); emulsifying agents (e.g., lecithin or acacia); non-aqueous vehicles (e.g., methyl or propyl-p-hydroxybenzoates or sorbic acid).

The composition may further include additional pharmaceutical ingredients to provide desirable characteristics, such as aesthetically pleasing qualities, improved taste, and the like, to otherwise render the dosage formulation more likely to be administered by the patient. Examples of desirable ingredients include, without limitation, penetration enhancers, colorants, flavorings agents, solvents and co-solvents, coating agents, direct compression excipients, disintegrants, glidants, lubricants, polishing agents, suspending agents, sweetening agents, anti-adherents, binders, and diluents. The ingredients may also include preservatives, emulsifying agents, antioxidants, plasticizers, surfactants, tonicity agents, viscosity increasing agents and combinations thereof. Examples of useful additives include, without limitation, propylene glycol, polyethylene glycol, orange, cherry, mint, and strawberry flavors and other commonly utilized ingredients.

The term "subject in need thereof" refers to any animal in need of relief from the symptoms of opiate addiction withdrawal, migraine, neuropathic pain, or conditions that can be treated with lofexidine. Preferably, the subject is a mammal. More preferably, the subject is human.

This invention also includes pharmaceutical compositions, which contain as the active ingredient, one or more of the compounds of the subject invention above, associated with one or more pharmaceutically acceptable carriers or excipients. The excipient employed is typically one suitable for administration to human subjects or other mammals. In making the compositions of this invention, the active ingredient is usually mixed with an excipient or diluted by an excipient. When the excipient serves as a diluent, the excipient can be a solid, semi-solid, or liquid material, and can act as a vehicle, carrier or medium for the active ingredient.

The compositions of the invention can be formulated so as to provide a sustained release delivery of lofexidine. In another embodiment, the compositions of the present invention can be formulated to provide a mixture of fast/sustained release of lofexidine after administration to the patient by employing procedures known in the art.

The following examples are offered to illustrate this invention and are not to be construed in any way as limiting the scope of this invention.

Examples

The following Examples are provided to illustrate certain aspects of the present invention and to aid those of skill in the art in practicing the invention. These Examples are in no way to be considered to limit the scope of the invention in any manner.

EXAMPLE 1: **Sustained release oral formulation (Lot # AAL1) comprising lofexidine**

| Substance | Percent weight (%) | Weight (mg) |
|---|---|---|
| Lofexidine HCl | 1.6 | 3.2 |
| Hydroxypropylmethylcellulose | 30 | 60 |
| Carbopol 974P | 30 | 60 |
| Magnesium Stearate | 1.6 | 3.2 |
| Lactose anhydrous | 36.8 | 73.6 |
| | | |
| Total | 100 | 200 |

**EXAMPLE 2: Sustained release oral formulation (Lot # AAL2) comprising lofexidine**

| Substance | Percent weight (%) | Weight (mg) |
|---|---|---|
| Lofexidine HCl | 2 | 4.0 |
| Lactose anhydrous | 42 | 84 |
| Carbopol 974P | 15 | 30 |
| Calcium Phosphate | 11 | 22 |
| Stearic Acid | 2 | 4 |
| HPMC (Methocel F4M) | 28 | 56 |
| | | |
| Total | 100 | 200 |

**EXAMPLE 3: Sustained release oral formulation (Lot # AAL3) comprising lofexidine**

| Substance | Percent weight (%) | Weight (mg) |
|---|---|---|
| Lofexidine HCl | 2 | 4.0 |
| Lactose anhydrous | 40 | 150 |
| HPMC (Methocel F4M) | 56 | 90 |
| Magnesium Stearate | 2 | 45 |
| | | |
| Total | 100 | 200 |

Tablets of the above examples were manufactured using a wet granulation technique. In the wet granulation technique, lofexidine and the hydrophilic ingredients comprising the drug composition were mixed in a mixer to form a drug blend. Next, other ingredients comprising the part of the hydrophilic and hydrophobic polymers were dissolved in aqueous ethanolic solution to form a wet blend. Then, the prepared wet blend was slowly added to the drug blend with continual mixing in the mixer. The granulating fluid is added until wet granules are produced. The wet granules were then optionally forced through a predetermined screen onto oven trays. The blend was dried for 12 hours at 35°C oven. The dried granules were then sized through screen. Next, the lubricant such as magnesium stearate was added to the drug granulation. The powder blend for each lot was compressed into tablets using a single tablet compressing machine at 4000 psi for 15 minutes.

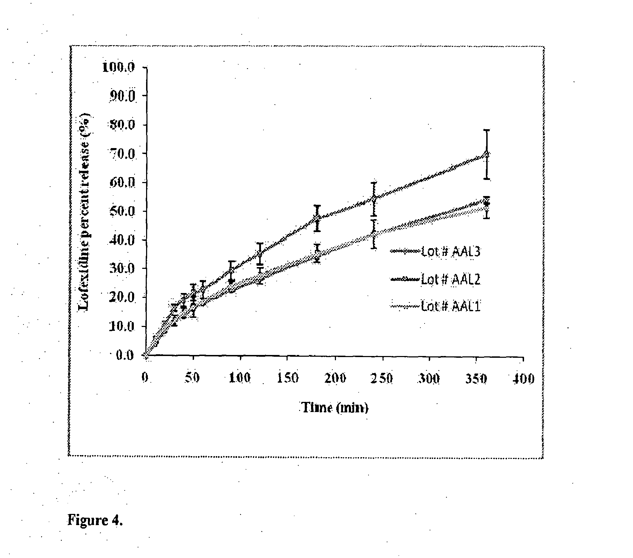

Three tablets from each of the formulations above were tested for *in vitro* lofexidine release using an Acid/Base dissolution (slightly modified USP 23/NF 18<711> Drug Release using Apparatus 2). Three dissolution vessels of a USP calibrated dissolution bath, equipped with shafts and paddles, were filled with 500 mL of 0.1N hydrochloric acid at 37.0C°. The bath and vessels were maintained at a temperature of 37.0 ± 0.5° C through out the 6 hr dissolution test. The paddles were set to rotate at 50 RPM and slowly lowered into the vessels. One tablet was then dropped into each vessel. At assigned time points of testing, 5 mL samples of dissolution solution were withdrawn from each vessel and filtered through a 10 micron polyethylene filter into HPLC vials and analyzed by UPLC/MS. In vitro lofexidine percentage release from the different tablets lots are shown in Figure 4.

All analytical procedures were performed using a Waters Acquity® Ultra UPLC/MS. An Acquity UPLC BEH Shield RP18 (2.1× 100 mm) column (Waters) was used to separate the chemical components at 40 °C. The flow rate was 0.3 mL/min of two mobile phases, (A) made of 5mM ammonium acetate (pH 4) and acetonitrile (90:10) and (B) of acetonitrile, 5mM ammonium acetate (pH 4) (90:10). The mass spectrometer was operated in the positive electrospray ionization (ESI) mode. The capillary voltage and cone voltage were maintained at 0.6 kV and 35 V, respectively. The source temperature and desolvation temperature were set at 100 and 350 °C, respectively. Nitrogen was used as both the cone gas (50 L/h) and the desolvation gas (700 L/h). Mass chromatograms and mass spectral data were acquired and processed by MassLynx software (Waters).

EXAMPLE 4: Modified release mixture of immediate release (IR) and sustained release (SR) formulation (Lot # AAL4) comprising **lofexidine**

| | IR Formulation | |
|---|---|---|
| Substance | Percent weight (%) | Weight (mg) |
| Lofexidine HCl | 0.4 | 0.2 |
| Microcrystalline cellulose | 62 | 31 |
| Sodium starch glycolate | 35 | 17.5 |
| Magnesium stearate | 2.6 | 1.3 |
| | | |
| Total | 100 | 50 |
| | | |

| | SR Formulation | |
|---|---|---|
| Substance | Percent weight (%) | Weight (mg) |
| Lofexidine HCl | 1.6 | 3.2 |
| Hydroxypropylmethylcellulose | 30 | 60 |
| Carbopol 974P | 30 | 60 |
| Magnesium Stearate | 1.6 | 3.2 |
| Lactose anhydrous | 36.8 | 73.6 |
| | | |
| Total | 100 | 200 |

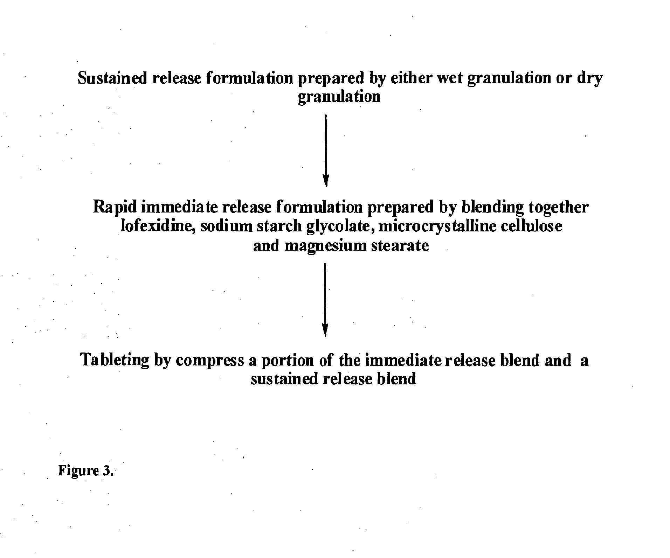

Tablets of the above example can be manufactured using either wet granulation or dry granulation techniques as described in Figure 3. The granules/blend from both formulations can be mixed and compressed into tablets using a single tablet compressing machine at 4000 psi for 15 minutes. Alternatively, the granules/blend can be filled in hard gelatin capsules suitable for oral use.

**Example 5: Transdermal gel formulation comprising lofexidine (Lot # AAL_G1)**

| | Gel Formulation | |
|---|---|---|
| Substance | Percent weight (%) | Weight (mg) |
| Lofexidine HCl | 2 | 20 |
| Hydroxypropylmethylcellulose | 5 | 50 |
| Propylene glycol 400 | 92 | 920 |
| Benzyl alcohol | 1 | 10 |
| | | |
| Total | 100 | 1000 |

**EXAMPLE 6: Transdermal delivery of lofexidine gel (Lot # AAL_G1) through porcine ear skin**

The skin permeation study was carried out using skin excised from porcine ears. Porcine ears were obtained fresh from a local slaughterhouse and were cleaned under cold running water. The whole skin was removed carefully from the outer region of the ear and separated from the underlying cartilage with a scalpel. Then it was allowed to dry for 30 minutes and afterwards it was wrapped into aluminum foil and stored at - 20°C until use.

In the experiment day, the skin was taken out of the freezer and thawed. After thawing it was wiped with a cotton ball wetted with phosphate buffer saline (BPS). Skin discs with suitable diameters were mounted onto Franz cells with a nominal area for diffusion of 1.7 cm² and a receptor volume of about 7 mL. The epidermal side was exposed to ambient conditions while the dermal side was bathed with PBS pH 7.4. The receptor fluid was kept at 32 ± 1°C and constant stirring was maintained by magnetic stirrer at 500 rpm. Care was taken to remove all air bubbles between the underside of the skin (dermis) and the receptor solution throughout the experiment. After conditioning and equilibration for 30 minutes, the gel formulation (Lot # AAL_G1) was applied 100µg/cm² to the skin in the donor compartment of the dissolution cells. The donor compartment was closed securely. Samples were taken from the receptor fluid (200 µL) at predetermined time points and the withdrawn volume was replaced with the same volume of fresh equilibrated PBS buffer to maintain a constant volume. Samples were analyzed by the UPLC/MS analytical method described earlier and the skin permeation data were plotted as the cumulative amount of drug collected in the receiver compartment as a function of time (Figure 5).

**EXAMPLE 7: Transdermal delivery of lofexidine gel (Lot # AAL_G1) through porcine ear skin pretreated with microneedles** (150 micron)

The skin permeation study was carried out using skin excised from porcine ears as described earlier. After conditioning and equilibration for 30 minutes, the skin samples were removed from the Franz cells and fixed in a Petri dish, the skin samples were perforated with the microneedle patch or the dermorollers with microneedles length of 150 micron. Afterwards the skin samples were mounted back into Franz cell and the gel formulation (Lot# AAL_G1) was applied 100µg/cm² to the skin in the donor compartment of the dissolution cells. The donor compartment was closed securely. Samples were taken from the receptor fluid (200 µL) at predetermined time points and the withdrawn volume was replaced with the same volume of fresh equilibrated PBS buffer to maintain a constant volume. Samples were analyzed by the UPLC/MS analytical method described earlier and the skin permeation data were plotted as the cumulative amount of drug collected in the receiver compartment as a function of time (Figure 6).

While this invention has been described as having a preferred embodiment, it is understood that the invention is not limited to the illustrated and described features. To the contrary, the invention is capable of further modifications, uses, and/or adaptations following the general principles of the invention and therefore includes such departures from the present disclosure as come within the known or customary practice in the art to which the invention pertains, and as may be applied to the central features set forth above, and which fall within the scope of the appended claims.

It would be obvious to those skilled in the art that modifications or variations may be made to the preferred embodiment described herein without departing from the novel teachings of the present invention. All such modifications and variations are intended to be incorporated herein and within the scope of the claims.

## Claims

1. A sustained release composition comprising lofexidine for use in oral administration.

2. The composition of claim 1, comprising a population of beads selected from the group consisting of immediate-release beads, enteric-release beads, and sustained-release beads.

3. The composition of claim 1 comprising lofexidine and about 10 to about 90% binder; about 1 to about 50% hydrophilic polymer, about 0.1 to about 50% hydrophobic polymer, about 0.1 to about 2% lubricant and glidant, and about 0.1 to about 1 % colorant.

4. The composition of claim 3, comprising about 30 to about 50% lactose as the binder; about 1 to 2.5% hydroxypropylmethylcellulose, as the hydrophilic polymer; about 2 to about 50 % acrylic resin as the hydrophobic polymer, about 0.5 to about 1 % magnesium stearate as the lubricant and glidant, and about 0.1 to about 0.5% colorant.

5. The composition of claim 1, comprising a sustained release pharmaceutical compositions suitable for use in various forms, e.g., as pills, tablets, powders, granules, capsules, liquids, sprays, gels, syrups, slurries, suspensions and the like, in bulk or unit dosage forms, for oral ingestion by a subject to be treated.

6. The composition of claim 1, which is prepared in a microencapsulated, liposomal, or nanoparticle form.

7. The composition of claim 1, wherein the sustained release composition further comprises an additional active agent.

8. The composition of claim 7, wherein the additional active agent is selected from an opioid, an opioid antagonist, a sedative or hypnotic, an anxiolytic, an antihistamine, a muscle relaxant, a cannabinoid.

9. The composition of claim 8, wherein the active agent is an opioid antagonist which comprises 7- benzylidenenaltrexone, beta-funaltrexamine, buprenorphine, butorphanol, chlornaltrexamine, clocinnamox, connective tissue-activating peptide, cyclazocine, diprenorphine, ICI 154129, levallorphan, meptazinol, methylnaltrexone, N,N- diallyl-tyrosyl-alpha-aminoisobutyric acid- phenylalanyl-leucine, nalbuphine, nalmefene, nalorphine, naloxone, naltrexone, or naltrindole, or mixtures or combinations thereof.

10. The method of claim 8, wherein the active agent is cannabinoid marinol.
